# EUROPEAN PATENT APPLICATION

(11) **EP 1 350 538 A1**
(43) Date of publication of application: **08.10.2003**
(21) Application number: 03075801.5
(22) Date of filing: 19.03.2003
(51) Int. Cl.: A61M 25/06

(54) **Needle assembly**

(30) Priority: 19.03.2002 US 365702 P
(71) Applicant: Becton, Dickinson and Company, Franklin Lakes, New Jersey 07417 (US)
(72) Inventor: Wilkinson, Bradley, North Haledon, NJ 10312 (US); Niermann, Volker, Bound Brook, NJ 08805 (US); Crawford, Jamieson, New york, NY 10025 (US)
(74) Representative: 't Jong, Bastiaan Jacob

(57) **Abstract**

A shieldable needle assembly provided as part of a blood collection set includes a needle cannula (22) having a proximal end and a distal end with a puncture tip (28). The needle assembly has a hub member (30) supporting the proximal end of the needle cannula (22) and a shield member (40) having a housing defining a central bore (44), which is in axial alignment with the hub member (30). The shield member (40) extends co-axially about the needle cannula (22) and is movable between a retracted position in which the puncture tip (28) of the needle cannula is exposed, and an extended position covering the puncture tip (28) of the needle cannula. An extendable member (70,100) connects the hub (30) and shield members (40). A compression spring (160) is further connected between the hub (30) and shield members (40) for moving the shield member to the extended position. A latch assembly provides releasable engagement between the hub and shield members.

## Description

### CROSS REFERENCE TO RELATED APPLICATIONS

This application claims the benefit of U.S. Provisional Application No. 60/365,702 filed March 19, 2002.

### BACKGROUND OF THE INVENTION

### 1. Field of the Invention

The present invention relates to blood collection sets for safe and convenient handling and disposal of medical needles and, more particularly, a disposable blood collection set including a passively actuated shieldable needle assembly.

### 2. Description of Related Art

Disposable medical devices having medical needles are used for administering medication or withdrawing fluid from the body of a patient. Such disposable medical devices typically include blood collecting needles, fluid handling needles, and assemblies thereof. Current medical practice requires that fluid containers and needle assemblies used in such devices be inexpensive and readily disposable. Consequently, existing blood collection devices typically employ some form of durable, reusable holder on which detachable and disposable medical needles and fluid collection tubes may be mounted. A blood collection device of this nature may be assembled prior to use and then disassembled after use. Thus, these blood collection devices allow repeated use of a relatively expensive holder upon replacement of relatively inexpensive medical needles and/or fluid collection tubes. In addition to reducing the cost of collecting blood specimens, these blood collection devices help minimize the production of hazardous waste material.

A blood collection device or intravenous "IV" infusion device typically includes a needle cannula having a proximal end, a pointed distal end, and a lumen extending therebetween. The proximal end of the needle cannula is mounted in a plastic hub defining a central passage that communicates with the lumen extending through the needle cannula. A thin, flexible thermoplastic tube is connected to the hub and communicates with the lumen of the needle cannula. The end of the plastic tube remote from the needle cannula may include a fixture for connecting the needle cannula to a blood collection tube or other receptacle. The specific construction of the fixture will depend upon the characteristics of the receptacle to which the fixture is to be connected.

In order to reduce the risk of incurring an accidental needle-stick wound, protection of used needle cannulas becomes important. With concern about infection and transmission of diseases, methods and devices to enclose or cover the used needle cannula have become very important and in great demand in the medical field. For example, needle assemblies commonly employ a safety shield that can be moved into shielding engagement with a used needle cannula to minimize risk of an accidental needle-stick.

Some needle safety shields are referred to as "tip guards" and include a small rigid guard that may be telescoped along the length of the needle cannula and extended over the pointed distal end of the needle cannula for protection.

Such conventional tip guards may include some form of tether for limiting the travel of the tip guard to the length of the needle cannula. An example of the foregoing is disclosed by U.S. Patent No. 5,176,655 to McCormick et al. The McCormick et al. patent discloses the use of flexible loop-like straps for limiting the distal movement of a tip guard.

Needle shields that incorporate movable tip guards are typically manually actuated. For example, U.S. Patent Nos. Re 36,447 and Re 36,398, both to Byrne et al., disclose a safety device for a hypodermic needle that includes a plastic sheath, which is used to cover the puncture tip of the needle. The plastic sheath incorporates a thumb guard, which the user of the safety device may grasp to move the plastic sheath to a position covering the puncture tip of the needle cannula. U.S. Patent No. 5,951,525 to Thorne et al. discloses a manually operated safety needle apparatus that includes two pairs of opposed legs adapted to move the tip guard of the apparatus to a position covering the used needle cannula. U.S. Patent Nos. 5,562,637 and 5,562,636, both to Utterburg, disclose a rectangular needle protector sheath for use with a needle cannula that may be extended over the needle cannula after its use.

Other prior art devices, such as those disclosed by U.S. Patent Nos. 5,290,264 to Utterberg and 5,192,275 to Burns provide "grippable" members attached to the tip guards to facilitate moving the tip guards to a position covering the puncture tip of a needle cannula. In addition to providing gripping members for moving the tip guards, prior art devices in this area often include flexible wings, which are used as means for securing the needle assemblies to the body of a patient during a medical procedure. Examples of "winged" needle assemblies may be found in U.S. Patent Nos. 5,120,320 to Fayngold; and 5,154,699; 5,088,982; and 5,085,639 all to Ryan. Other prior art in this area includes U.S. Patent Nos. 5,266,072 and 5,112,311, both to Utterberg et al., which also disclose guarded winged needle assemblies.

Conventional shields and tip guards, such as those discussed hereinabove, often further require extensive mechanics for positioning the shield or tip guard over the needle cannula. This results in complex arrangements that are costly to manufacture and assemble. Additionally, operation of the needle assemblies to move the tip guard into the proper position over the pointed distal end of the needle cannula requires substantial manual manipulation by the user of the device, exposing the user to potential needle-stick wounds.

In view of the foregoing, a need exists for a blood collection set including a shieldable needle assembly that achieves secure and effective shielding of a used needle cannula while requiring minimal manual input from the user of the needle assembly, and which is simple and inexpensive to manufacture.

### SUMMARY OF THE INVENTION

The present invention is directed to a blood collection set that includes a shieldable needle assembly. The blood collection set generally includes a fixture for connecting the blood collection set to a receptacle, a flexible tube connected to the fixture, and the shieldable needle assembly. The flexible tube has opposed first and second ends, with the first end of the flexible tube connected to the fixture. The needle assembly used in the blood collection set generally includes a needle cannula having a proximal end and a distal end with a puncture tip. The proximal end of the needle cannula is connected to a hub member, which supports the proximal end of the needle cannula. The distal end of the needle cannula projects outward from the hub member. The needle cannula also defines a lumen in fluid communication with the flexible tube and the fixture. The needle assembly further includes a shield member having a housing defining a central bore. The shield member is in axial alignment with the hub member and extends coaxially about the needle cannula. The shield member is movable between a retracted position in which the puncture tip of the needle is exposed, and an extended position in which the shield member covers the puncture tip of the needle cannula. An extendable member is connected between the hub member and the shield member. A compression spring is operatively connected between the hub member and the shield member. A latch assembly provides releasable engagement between the hub member and the shield member. When the latch assembly is in engagement between the hub member and the shield member, the shield member is in the retracted position. When the latch assembly is released of engagement between the hub member and the shield member, the shield member is moved to the extended position through forces exerted by the compression spring, thereby covering the puncture tip of the needle cannula.

The latch assembly may include a pivoting latch member located on the hub member or the shield member. The latch member is generally configured to releaseably engage with a catch member located on the other of the hub member and shield member. Thus, when the latch member is pivoted out of engagement with the catch member, the latch assembly is released of engagement between the hub member and the shield member, thereby causing the shield member to be moved to the extended position by the compression spring.

The shield member may include a pair of butterfly wings extending laterally from opposing sides of the housing of the shield member. The housing of the shield member may further include a dorsal wing located between the butterfly wings. Similarly, the hub member may include a pair of butterfly wings, and a dorsal wing located between the butterfly wings.

The compression spring may be located co-axially about the needle cannula. Alternatively, the compression spring may be located adjacent the needle cannula, extending between the hub member and the shield member.

The shield member may further include a tip guard for protectively surrounding the puncture tip of the needle cannula when the shield member is moved to the extended position. Alternatively, the tip guard may be externally attached to the housing of the shield member.

The extendable member may include a pair of folding legs connected by a hinged knee joint. Preferably, one leg of the pair of legs is hingedly connected to the hub member and the other leg of the pair of legs is hingedly connected to the shield member. The knee joint may further include a fingerplate to enable the user to manipulate the assembly.

The needle assembly may include a locking assembly for locking the shield member in the extended position. The locking assembly may include a pair of locking members extending from the fingerplate toward the needle cannula and configured to engage the needle cannula such that when the shield member is moved to the extended position by the compression spring, the locking to secure the shield member in the extended position.

The needle assembly may include a second extendable member connecting the hub and shield members and located opposite from the first extendable member. Each of the extendable members may include a pair of folding legs connected by a hinged knee joint, with one leg of the pair of legs for each extendable member hingedly connected to the hub member and the other leg of the pair of legs for each extendable member hingedly connected to the shield member. The knee joint for each extendable member may include a fingerplate. A locking assembly may be provided for locking the shield member in the extended position, with the extendable members extending therebetween. The locking assembly may include a locking member extending from one of the fingerplates toward the opposing fingerplate. The locking member is preferably configured to engage a second locking member extending from the opposing fingerplate, such that when the shield member is moved to the extended position by the compression spring, the locking members engage to secure the shield member in the extended position.

The hub member and shield member may each include a pair of pivoting butterfly wings extending laterally from opposing sides thereof. The latch assembly may be located to provide releasable connections between the butterfly wings extending from the hub and shield members. For example, the latch assembly may include a pair of latch members located, respectively, on the wings extending from the shield member and be configured to releasably engage with a second pair of latch members located, respectively, on the wings extending from the hub member. When the respective pairs of latch members are engaged, the shield member is maintained in the retracted position. When one of the pairs of wings extending from the hub and shield members is pivoted, the engaged latch members are disengaged and the shield member is moved to the extended position by the compression spring, thereby covering the puncture tip of the needle cannula.

Furthermore, the present invention is directed to a method of automatically placing a shieldable needle assembly into a safety state in which a puncture tip of a needle cannula is covered by the needle assembly. The method may generally include the steps of: providing a shieldable needle assembly as generally described hereinabove; using the needle assembly in a medical procedure; and pivoting the latch member of the latch assembly out of engagement with the catch member of the latch assembly such that the shield member is moved to the extended position through the force of the compression spring, thereby covering the puncture tip of the needle cannula.

Further details and advantages of the present invention will become apparent from the following detailed description when read in conjunction with the drawings.

### DESCRIPTION OF THE DRAWINGS

FIG. 1 is a perspective view of a blood collection set and a shieldable needle assembly in accordance with a first embodiment of the present invention showing the needle assembly in a retracted position;
FIG. 2 is a perspective view of the blood collection set of FIG. 1 showing the needle assembly in a partially extended position;
FIG. 3 is a perspective of the blood collection set of FIG. 1 showing the needle assembly in a fully extended and locked position;
FIG. 4 is a cross-sectional view taken along line IV-IV of FIG. 1;
FIG. 5 is a cross-sectional view taken along line V-V of FIG. 1;
FIG. 6 is a side view of a tip guard portion of the needle assembly of FIG. 1;
FIG. 7 is a side view of the tip guard portion of FIG. 6 showing the tip guard portion covering a distal end of the needle assembly;
FIG. 8 is a perspective view of the blood collection set and shieldable needle assembly in accordance with a second embodiment of the present invention showing the needle assembly in a retracted position;
FIG. 9 is a perspective view of the blood collection set of FIG. 8 showing the needle assembly in a partially extended position;
FIG. 10 is a cross-sectional view taken along line X-X of FIG. 8;
FIG. 11 is a perspective view of the blood collection set of FIG. 1 wherein the needle assembly incorporates a modified tip guard portion in accordance with the present invention;
FIG. 12 is a perspective view of the blood collection set of FIG. 11 showing the needle assembly in a partially extended position;
FIG. 13 is a cross-sectional view taken along line XIII-XIII in FIG. 11;
FIG. 14 is a cross-sectional view taken along line XIV-XIV in FIG 12;
FIG. 15 is a perspective view of the blood collection set and shieldable needle assembly in accordance with a third embodiment of the present invention and showing the needle assembly in a retracted position;
FIG. 16 is a perspective view of the blood collection set of FIG. 15 showing the needle assembly in a partially extended position;
FIG. 17 is a cross-sectional view taken along line XVII-XVII in FIG. 15;
FIG. 18 is a cross-sectional view taken along line XVIII-XVIII in FIG. 15;
FIG. 19 is a perspective view of the shieldable needle assembly in accordance with a fourth embodiment of the present invention; and
FIG. 20 is a perspective view of the needle assembly of FIG. 19 including the modified tip guard portion of FIG. 11.

### DETAILED DESCRIPTION

Referring to the drawings in which like reference characters refer to like parts throughout the several views thereof, the present invention is generally described in terms of a blood collection set and related features, and encompasses such a blood collection set as well as a shieldable needle assembly for use in such a blood collection set.

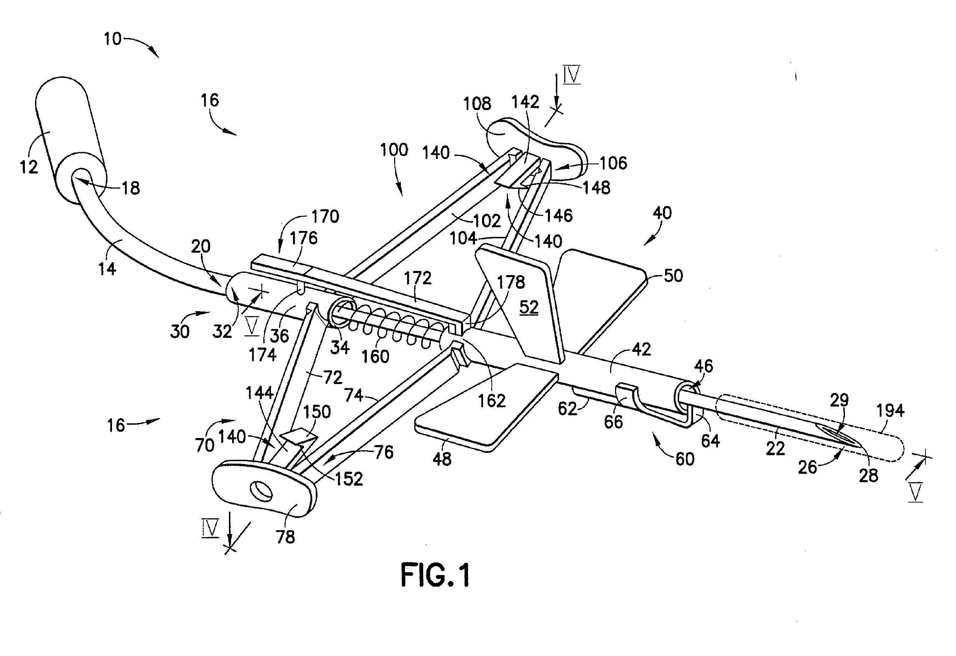

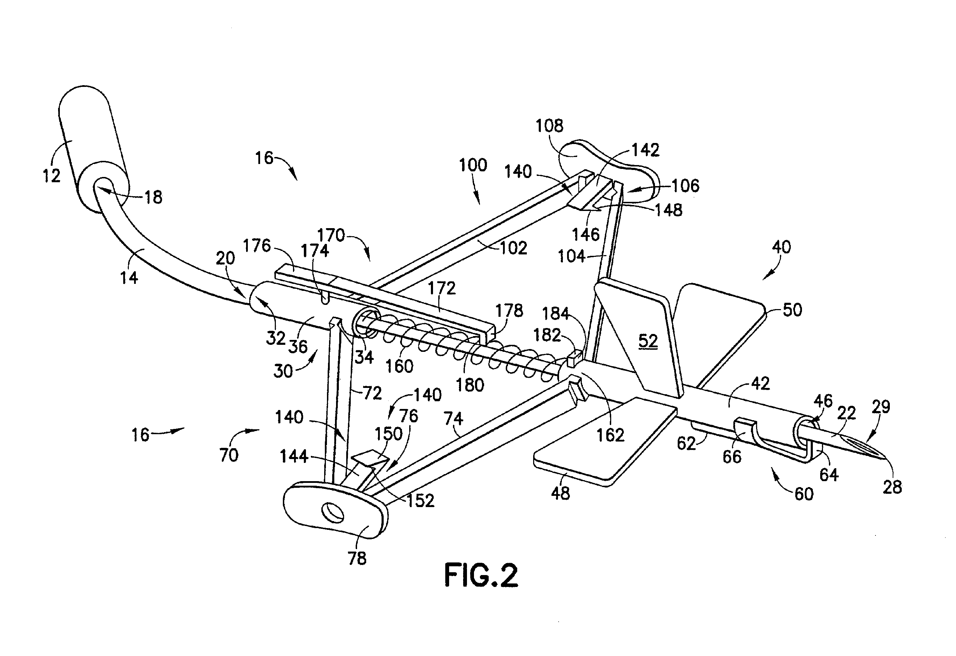

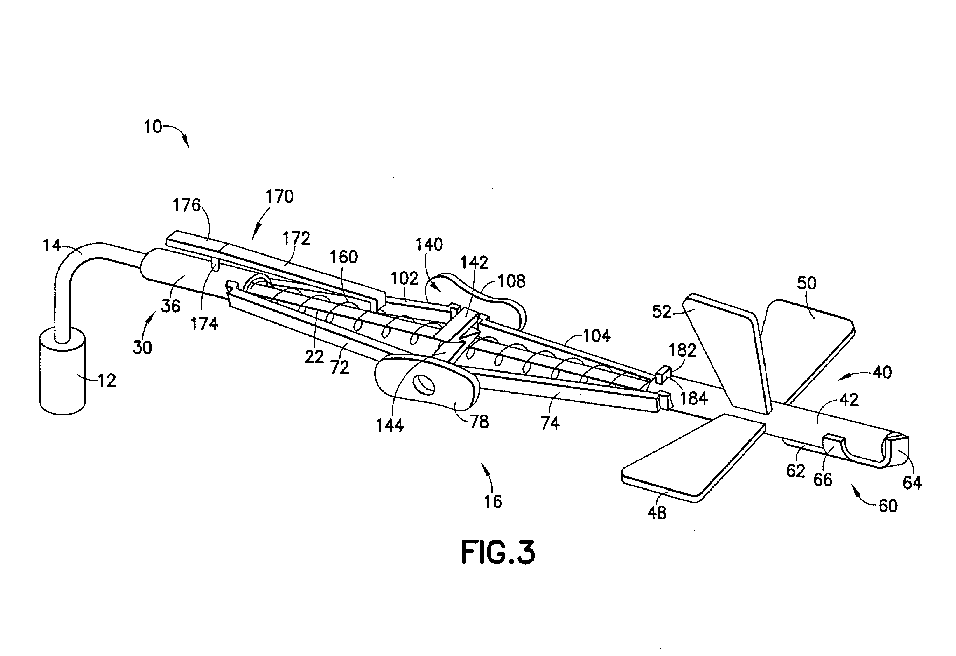

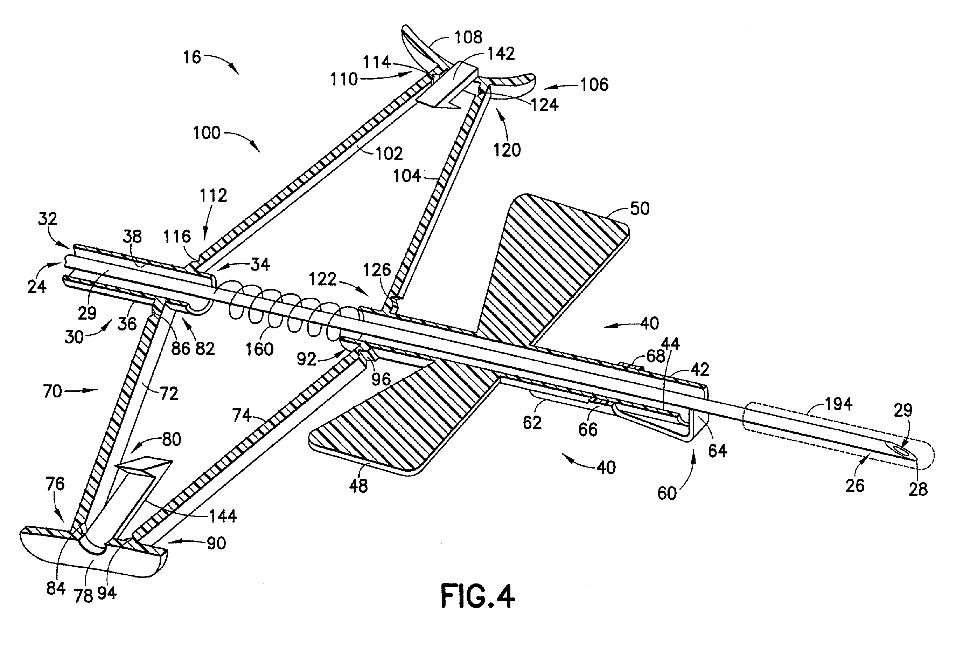

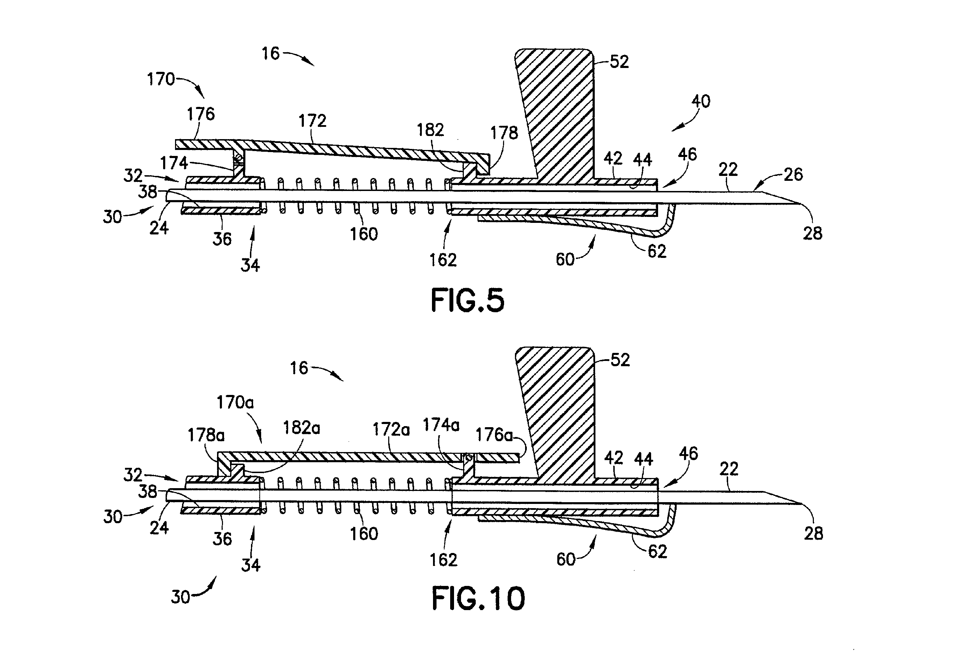

Referring to FIGS. 1-5, a blood collection set **10** in accordance with the present invention includes a fixture **12** for connecting the blood collection set **10** to a receptacle (not shown), a flexible tube **14**, and a shieldable needle assembly **16**. The flexible tube **14** has a first end **18** and a second end **20**. The first end **18** of the flexible tube **14** is connected to the fixture **12**. The first end **18** of the flexible tube **14** may be connected to the fixture **12** by means customary in the art.

The shieldable needle assembly **16** of the blood collection set **10** includes a needle cannula **22**. The needle cannula **22** has a proximal end **24** and an opposing distal end **26**. The needle cannula **22** defines a lumen **29** extending through the needle cannula **22** from the proximal end **24** to the distal end **26**. The distal end **26** of the needle cannula **22** is beveled to define a sharp puncture tip **28**, such as an intravenous (IV) puncture tip. The puncture tip **28** is provided for insertion into a patient's blood vessel, such as a vein, and is, therefore, designed to provide ease of insertion and minimal discomfort during venipuncture.

The shieldable needle assembly **16** of the blood collection set **10** includes a hub member **30**, a shield member **40**, and at least one extendable member **70** and, preferably, a pair of extendable members **70**, **100** connecting the hub member **30** and the shield member **40**. The hub member **30**, shield member **40**, and extendable member **70** may be integrally formed as a unitary body, which is desirably molded from a thermoplastic material. However, the hub member **30**, shield member **40**, and extendable member **70** may be separate parts, which are preferably molded from thermoplastic material.

The hub member **30** has a proximal end **32** and a distal end **34** and is generally defined as a rigid tubular wall **36** extending from the proximal end **32** to the distal end **34**. The tubular wall **36** defines an internal passage **38** extending from the proximal end **32** to the distal end **34**. The hub member **30** is adapted to support the proximal end **24** of the needle cannula **22**. In particular, the needle cannula **22** is positioned within the internal passage **38** defined by the hub member **30** and extends outward from the distal end **34** of the hub member **30**. Preferably, the needle cannula **22** and hub member **30** are formed as separate parts that are fixedly attached and secured through an appropriate medical grade adhesive, by direct mechanical attachment, or other similar means.

The shield member **40** is movable along the needle cannula **22** between a first proximal position or retracted position located adjacent the hub member **30**, and a second distal position or extended position covering the puncture tip **28** of the needle cannula **22**. The shield member **40** includes a housing **42** defining a central bore **44** having a distal opening **46** from which the puncture tip **28** extends when the shield member **40** is in the retracted position. The housing **42** of the shield member **40** extends coaxially about the needle cannula **22** and is in axial alignment with the internal passage **38** defined by the tubular wall **36** of the hub member **30**. The shield member **40** further includes a pair of stabilizers in the form of wings **48**, **50** that extend laterally from the housing **42** at opposing sides thereof. The lateral wings **48**, **50** provide a butterfly-type wing assembly useful for positioning and placement of the needle assembly **16** and blood collection set **10** during a blood collection procedure. The shield member **40** may further include a dorsal wing **52** extending from the housing **42** and located between the lateral wings **48, 50**. The dorsal wing **52** is preferably symmetrically positioned on the housing **42** between the lateral wings **48, 50**.

Optionally, a needle tip guard **60** is attached to the housing **42** of the shield member **40** distally forward of the lateral wings **48**, **50** and dorsal wing **52**. The tip guard **60** is provided to automatically cover the distal opening **46** of the central bore **44** of the housing **42** when the shield member **40** is moved to the fully extended position covering the puncture tip **28** of the needle cannula **22**. The tip guard **60** is provided as a curved leaf spring of metal or the like having an axially extending spring leg **62**, a generally right-angled blocking plate **64**, and a pair of clip legs **66**, **68** forming a gripping collar for securely holding the tip guard **60** onto the housing **42** of the shield member **40**. Operation of the tip guard **60** shown in FIGS. 1-5 will be discussed hereinafter.

As stated previously, the hub member **30** and shield member **40** are connected by at least one extendable member **70** and, preferably, a pair of extendable members **70**, **100**. The first extendable member **70** is comprised of a pair of folding legs, which include a first or proximal leg **72** and a second or distal leg **74**. The first and second legs **72**, **74** are connected by a hinged knee joint **76** that includes a fingerplate **78**. The first leg **72** of the pair of folding legs is hingedly connected to the hub member **30** and the second leg **74** of the pair of legs is hingedly connected to the shield member **40**. The first leg **72** includes a first end **80** and a second end **82**. The first end **80** of the first leg **72** is connected to the fingerplate **78** by a hinged connection **84**, and the second end **82** of the first leg **72** is connected to the hub member **30** by a hinged connection **86**. Likewise, the second leg **74** includes a first end **90** and a second end **92**. The first end **90** of the second leg **74** is connected by a hinged connection **94** to the fingerplate **78**, and the second end **92** of the second leg **74** is connected by a hinged connection **96** to the shield member **40**. Preferably, the fingerplate **78** of the knee joint **76** is concave to provide a convenient grasping location for the user of the needle assembly **16**.

Similarly, the second extendable member **100** is comprised of a pair of folding legs, which include a first or proximal leg **102** and a second or distal leg **104**. The first and second legs **102**, **104** are connected by a hinged knee joint **106** that includes a fingerplate **108**. The first leg **102** of the pair of folding legs is hingedly connected to the hub member **30** and the second leg **104** of the pair of legs is hingedly connected to the shield member **40**. The first leg **102** includes a first end **110** and a second end **112.** The first end **110** of the first leg **102** is connected to the fingerplate **106** by a hinged connection **114**, and the second end **112** of the first leg **102** is connected to the hub member **30** by a hinged connection **116**. Likewise, the second leg **104** includes a first end **120** and a second end **122**. The first end **120** of the second leg **104** is connected by a hinged connection **124** to the fingerplate **108**, and the second end **122** of the second leg **104** is connected by a hinged connection **126** to the shield member **40**. Preferably, the fingerplate **108** of the knee joint **106** is also concave to provide a convenient grasping location for the user of the needle assembly **16**. The respective folding legs **72**, **74** and **102**, **104** of the first and second extendable members **70**, **100** are configured to generally extend laterally along the lateral sides of the needle assembly **16** when the shield member **40** is moved to the extended position.

The needle assembly **16** further includes a locking assembly **140** located on the extendable members **70**, **100** for locking the shield member **40** in the extended position. In particular the locking assembly **140** is preferably integrally formed as part of the knee joints **76**, **106**. The locking assembly **140** is formed by a first locking member **142** and a second complimentary locking member **144**. The first locking member **142** extends from the inside surface of fingerplate **108** and extends toward opposing fingerplate **78**. The locking member **142** includes a sloped-leading edge **146** located opposite from a substantially planar locking edge **148**. The second locking member **144** extends from the inside surface of opposing fingerplate **78** and is configured to cooperate with (i.e., receive) the first locking member **142**. The locking catch member **144** also includes a sloped leading edge **150** located opposite from a substantially planar locking edge **152**, which is adapted to receive the locking edge **148** formed on the first locking member **142**. The operation of the locking assembly **140** will be discussed more fully hereinafter. It will be appreciated by those skilled in the art that the locations for the locking members **142**, **144** may be reversed.

The needle assembly **16** further includes a compression spring **160** operatively connected between the hub member **30** and shield member **40**. In particular, the compression spring **160** is connected between a proximal end **162** of the housing **42** of the shield member **40** and the distal end **34** of the hub member **30**. The compression spring **160** is mechanically attached, for example by fasteners, to the proximal end **162** of the housing **42** and the distal end **34** of the hub member **30**. Other equivalent means may be used to attach the compression spring **160** to the hub member **30** and shield member **40**, such as by an adhesive, are within the scope of the present invention. The compression spring **160** is preferably adapted to provide outward axial forces acting on the hub member **30** and shield member **40** that continuously bias the hub member **30** and shield member **40** away from each other (i.e., axially apart). The compression spring **160** preferably continues to apply axial forces biasing the hub member **30** and shield member **40** apart when the shield member **40** is moved to its fully extended position.

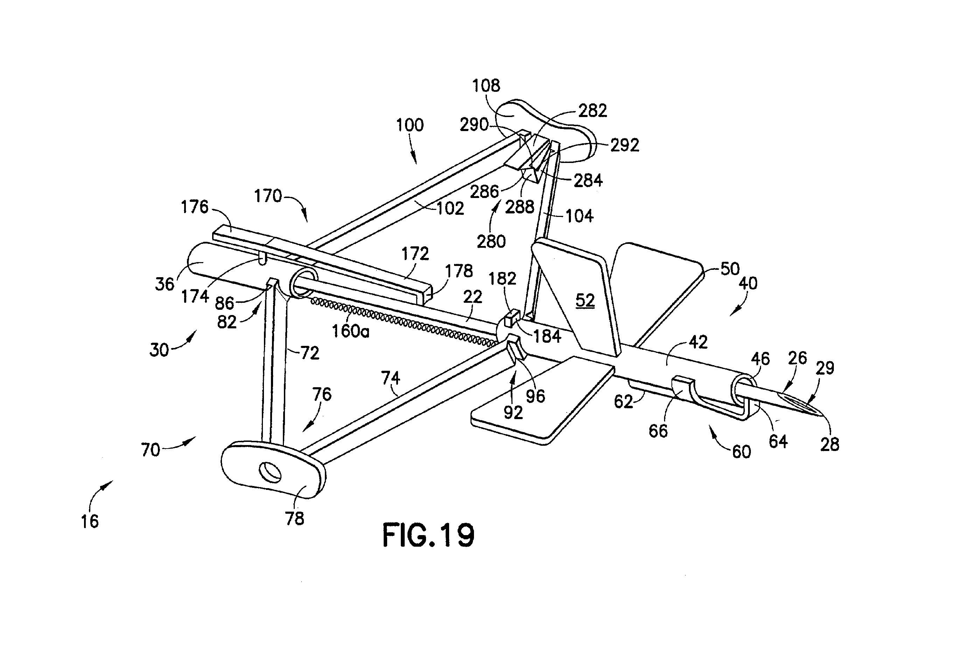

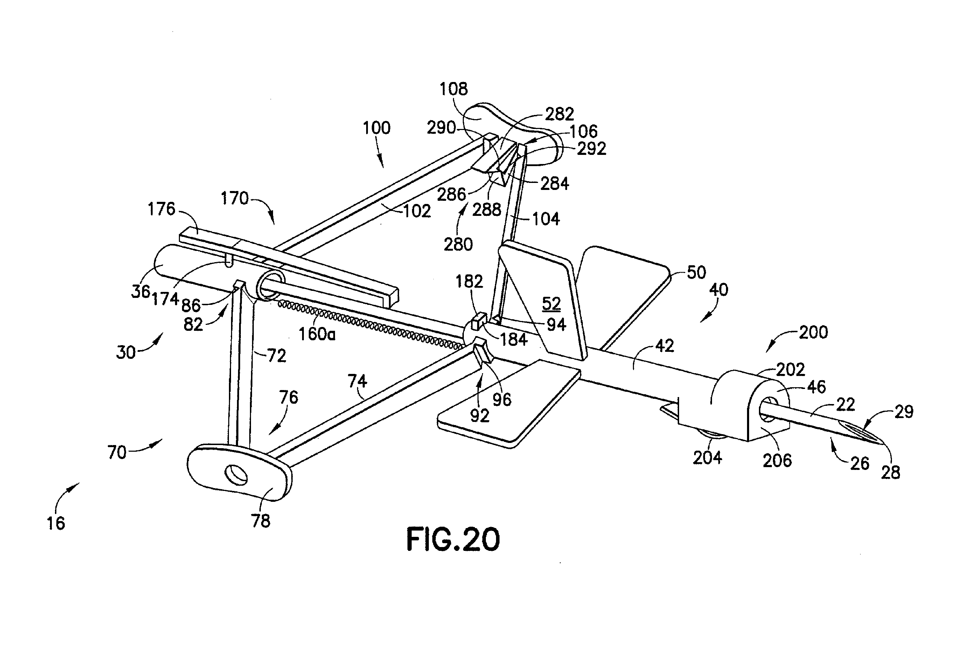

The compression spring **160** is located coaxially about the needle cannula **22**. Other orientations for the compression spring **160** are also envisioned by the present invention. One such alternative orientation is shown in FIGS. 19 and 20, discussed hereinafter, in which the compression spring **160** is located adjacent and extends laterally along the needle cannula **22**.

The needle assembly **16** further includes a latch assembly **170** configured to provide releasable engagement between the hub member **30** and the shield member **40**. The latch assembly **170** is used to prevent the compression spring **160** from moving the shield member **40** to the extended position until the latch assembly **170** is released of engagement between the hub member **30** and the shield member **40**. Once the latch assembly **170** is released of engagement between the hub member **30** and the shield member **40**, the compression spring **160** provides all the necessary force to move the shield member **40** to the extended position. The latch assembly **170** includes a pivoting latch member **172** located on a top surface of the hub member **30** and extending toward the shield member **40**. The pivoting latch member **172** includes a pivot support **174** about which the latch member **172** is configured to pivot. The pivotal connection between the latch member **172** and the pivot support **174** may be provided, for example, by a hinge. The latch member **172** includes a fingerplate **176** for pivoting the latch member **172** about the pivot support **174**. The latch member **172** has a transversely extending edge **178** at a distal end thereof. A locking edge **180** is located opposite from the transversely extending edge **178**. The latch member **172** is configured to cooperate with a rectangular-shaped catch member **182** located on the shield member **40**. The catch member **182** has a locking edge **184** at a distal end thereof, which is engaged by the locking edge **180** of the latch member **172**. Once the latch member **172** is in engagement with the catch member **182**, the compression spring **160** will be restrained from moving the shield member **40** to the extended position. The locations for the latch member **172** and the catch member **182** may be reversed, as will be appreciated by those skilled in the art.

A removable, protective needle cover **194** may be used to cover the distal end **26** of the needle cannula **22** and, more particularly, the puncture tip **28** of the needle cannula **22**. The needle cover **194** is preferably positioned over the puncture tip **28** of the needle cannula **22** in a pre-use state of the needle assembly **16**, wherein the shield member **40** is in the retracted position and maintained in the retracted position by the latch assembly **170**. The needle cover **194** is preferably an inexpensive, elongated plastic needle cover such as those commonly used as a needle protector in the medical field.

With the basic structure of the blood collection set **10** and needle assembly **16** now described, operation of the blood collection set **10** and needle assembly **16** will be described with reference to FIGS. 1-7. The needle assembly **16** is preferably provided with the shield member **40** in the retracted state and the needle cover **194** positioned over the distal end **26** of the needle cannula **22**. The shield member **40** is held in the retracted position by the latch assembly **170**. In particular, the latch member **172** extending from the hub member **30** is in engagement with the catch member **182** located on the shield member **40**. The respective locking edges **180**, **184** of the latch member **172** and catch member **182** maintain the engagement between the latch member **172** and the catch member **182**, as well as restrain the compression spring **160** from separating the hub member **30** and the shield member **40**. A blood collection tube may then be affixed to the fixture **12** located at the first end **18** of the flexible tube **14**.

To use the blood collection set **10** and needle assembly **16** in a medical procedure, the user of the blood collection set **10** will first sterilize the intended area of puncture on the patient's body and remove the needle cover **194** from the distal end **26** of the needle cannula **22**. The user of the needle assembly **16** may then grasp the lateral wings **48**, **50** and the dorsal wing **52** to assist in positioning the needle assembly **16** at the intended area of puncture on the patient's body. The lateral wings **48**, **50** and the dorsal wing **52** are preferably made flexible so that they may be folded together to provide a convenient handle for manipulating the needle assembly **16**. Once the puncture tip **28** of the needle cannula **22** is inserted into a blood vessel in the patient's body (i.e., venipuncture), the user may spread the lateral wings **48**, **50** flat onto the patient's body to maintain the positioning and placement of the needle assembly **16** during the blood collection procedure or other medical procedure. The lateral wings **48**, **50** in this configuration will also provide a barrier between the needle cannula **22** and the user's fingertips, which will help prevent an accidental needle-stick should the needle cannula **22** inadvertently retract from the site of insertion. The user of the needle assembly **16** may also grasp the fingerplates **78**, **108** attached to the extendable members **70**, **100** with his or her free hand to further assist in positioning and placing the needle assembly **16** at the intended site of insertion into the patient's body.

After completing venipuncture, the user of the needle assembly **16** will typically collect one or more blood samples by attaching one or more blood collection tubes (not shown) to the fixture **12**. Once the blood collection step is completed, the user of the blood collection set **10** and needle assembly **16** may then actuate the needle assembly **16** to move the shield member **40** to a partially extended position. The shield member **40** may be moved partially to the extended position while the needle cannula **22** remains in the patient's body, which significantly reduces the probability of a needle-stick wound from occurring. To actuate the needle assembly **16**, the user of the needle assembly **16** pivots the latch member **172** located on the hub member **30** out of engagement with the catch member **182** located on the shield member **40** by pressing downward on the fingerplate **176**.

Once the user pivots the latch member **172** out of engagement with the catch member **182**, the latch assembly **170** is free of engagement between the hub member **30** and the shield member **40**, and the compression spring **160** is no longer restrained. Thus, the compression spring **160** automatically (i.e., passively) moves the shield member **40** to the extended position. The compression spring **160** has a spring constant and free length sufficient to move the shield member **40** to an at least partially extended position while the needle cannula **22** is in the patient's body without entirely removing the needle cannula **22** from the patient's body. The user may then completely withdraw the needle assembly **16** from the patient's body. Thereafter, the compression spring **160** provides sufficient outward axial force between the hub member **30** and the shield member **40** to move the shield member **40** to the extended position covering the puncture tip **28** of the needle cannula **22**.

In an alternative method of using the blood collection set **10** and needle assembly **16**, the user may elect to entirely withdraw the needle cannula **22** from the patient's body before actuating the needle assembly **16**. After completely withdrawing the needle cannula **22** from the patient's body, the user of the needle assembly **16** pivots the latch member **172** out of engagement with the catch member **182** by pressing downward on the fingerplate **176**. Once the latch assembly **170** is free of engagement between the hub member **30** and the shield member **40**, the compression spring **160** automatically (i.e., passively) biases the shield member **40** to the extended position covering the distal end **26** and, in particular, the puncture tip **28** of the needle cannula **22**.

In either method of operation discussed hereinabove, as the shield member **40** moves forward toward its extended position, the locking members **142**, **144** extending from the respective fingerplates **108**, **76** of the extendable members **70**, **100** begin to move toward one another. As the shield member **40** travels along the needle cannula **22** toward its extended position, the sloped leading edge **146** of the first locking member **142** slidably engages the sloped leading edge **150** of the second locking member **144**. As the shield member **40** reaches its fully extended position, the locking edge **148** of the first locking member **142** snaps into engagement with the locking edge **152** of the second locking member **144** to secure the shield member **40** in the extended position. Once the locking members **142**, **144** are in engagement, the extendable members **70**, **100** will be prevented from moving transversely away from the lateral sides of the needle assembly **16**. Thus, the locking assembly **140**, once engaged, prevents the re-emergence of the puncture tip **28** of the needle cannula **22** from the shield member **40**.

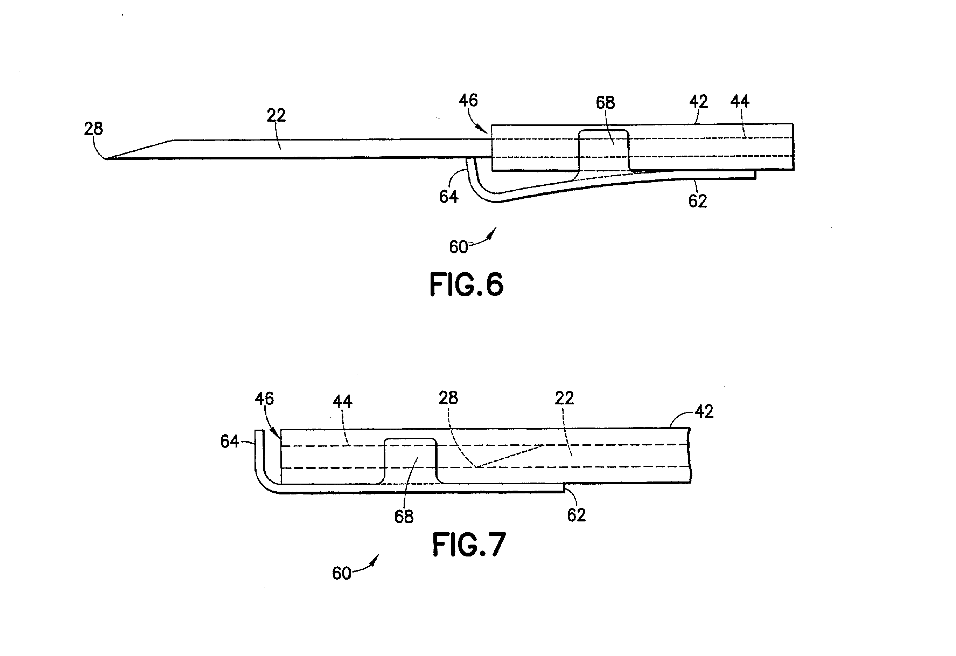

The tip guard **60** attached to the housing **42** of the shield member **40** will automatically cover the distal opening **46** to the central bore **44** once the needle cannula **22** is fully covered by the shield member **40**. As shown in FIGS. 6 and 7, when the shield member **40** is in the retracted position, the needle cannula **22** extends outward from the housing **42** of the shield member **40** and the blocking plate **64** of the tip guard **60** is biased into engagement with the needle cannula **22**. In particular, the spring leg **62** biases the blocking plate **64** into engagement with the needle cannula **22**. As the needle cannula **22** is covered by the shield member **40** upon disengagement of the latch assembly **170**, the blocking plate **64** slides along a bottom surface of the needle cannula **22** until the needle cannula **22** is fully covered by the shield member **40**. Once the blocking plate **64** is no longer in engagement with the needle cannula **22**, the axially extending spring leg **62** causes the blocking plate **64** to automatically extend over the distal opening **46** to the central bore **44** of the housing **42**. The tip guard **60** fully covers the distal opening **46** to the central bore **44** of the housing **42**, thereby preventing any re-emergence of the needle cannula **22** should the latching assembly **170** inadvertently become disengaged. With the blood collection set **10** and needle assembly **16** now placed in a safety state, the blood collection tube may be safely removed from the needle assembly **16**, and the needle assembly **16** disposed of as medical waste.

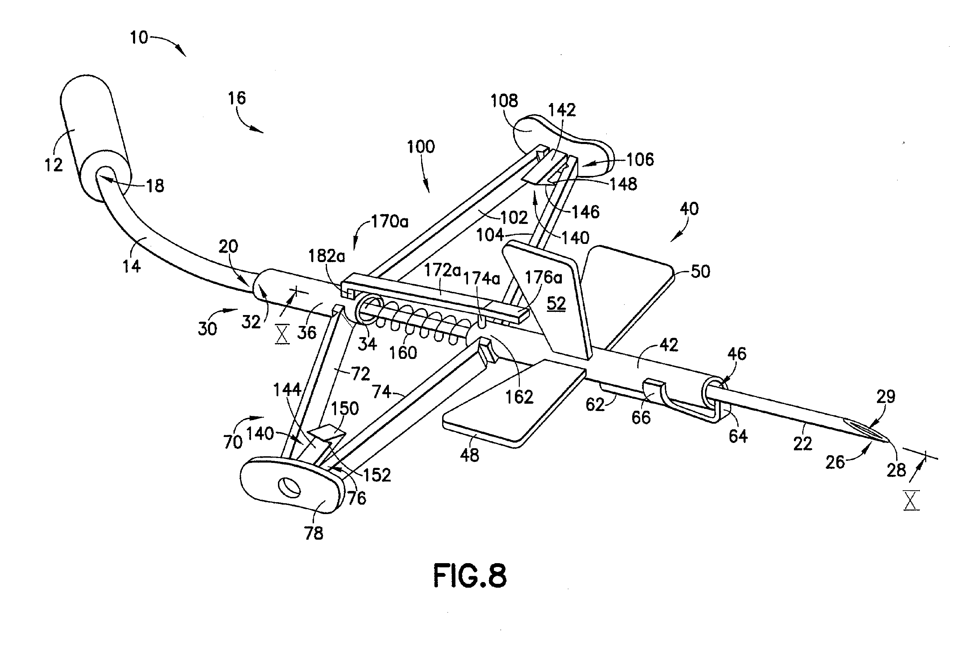

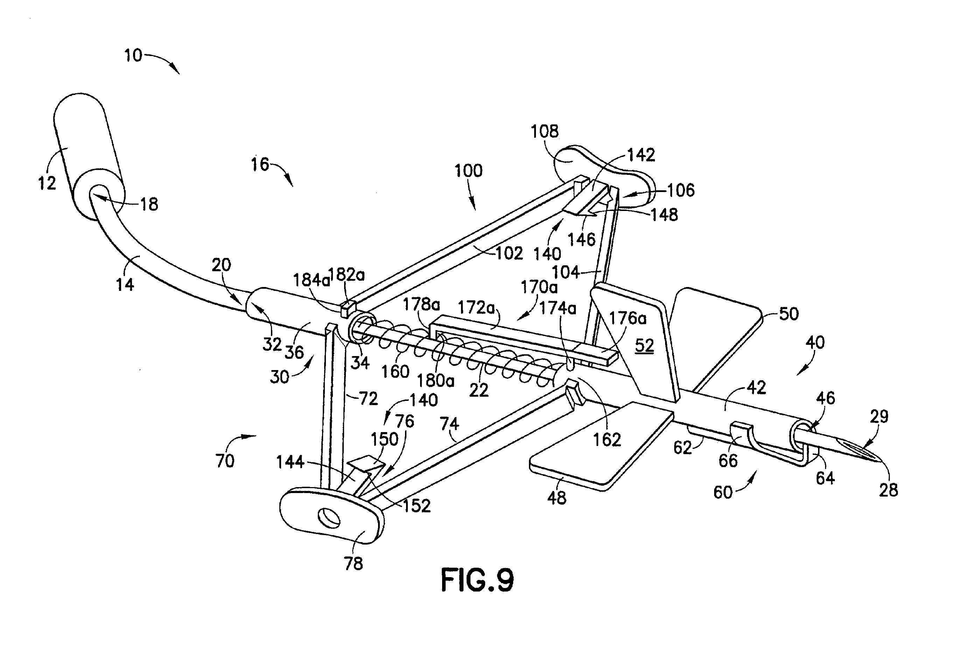

A modification to the blood collection set **10** and the needle assembly **16** as shown in FIGS. 1-5 will now be discussed with reference to FIGS. 8-10. The blood collection set **10** and needle assembly **16** shown in FIGS. 8-10 is substantially similar to the blood collection set **10** discussed in connection with FIGS. 1-5 with the modification that the locations for the latch member and the catch member of the locking assembly **170a** are now reversed. In particular, as shown in FIGS. 8-10, the latch member **172a**, pivot support **174a**, and fingerplate **176a** are now located on the top surface of the housing **42** of the shield member **40**, including transversely extending edge **178a** and locking edge **180a**. The catch member **182a** is now located on the top surface of the hub member **30.** The remaining aspects and operation of the blood collection set **10** and needle assembly **16** of FIGS. 8-10 remain the same as the blood collection set **10** and needle assembly **16** discussed previously in connection with FIGS. 1-5.

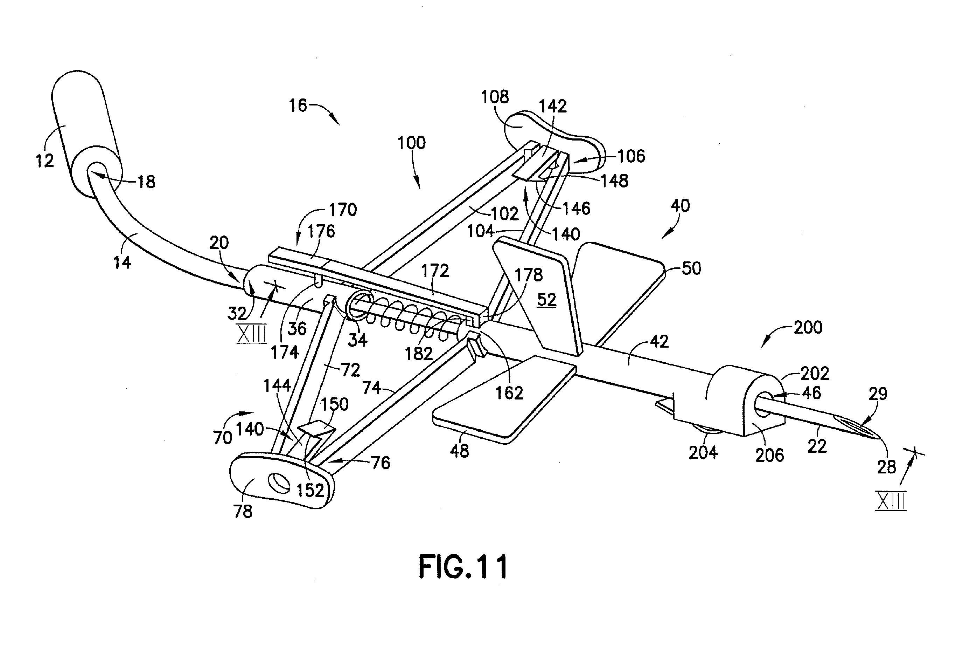

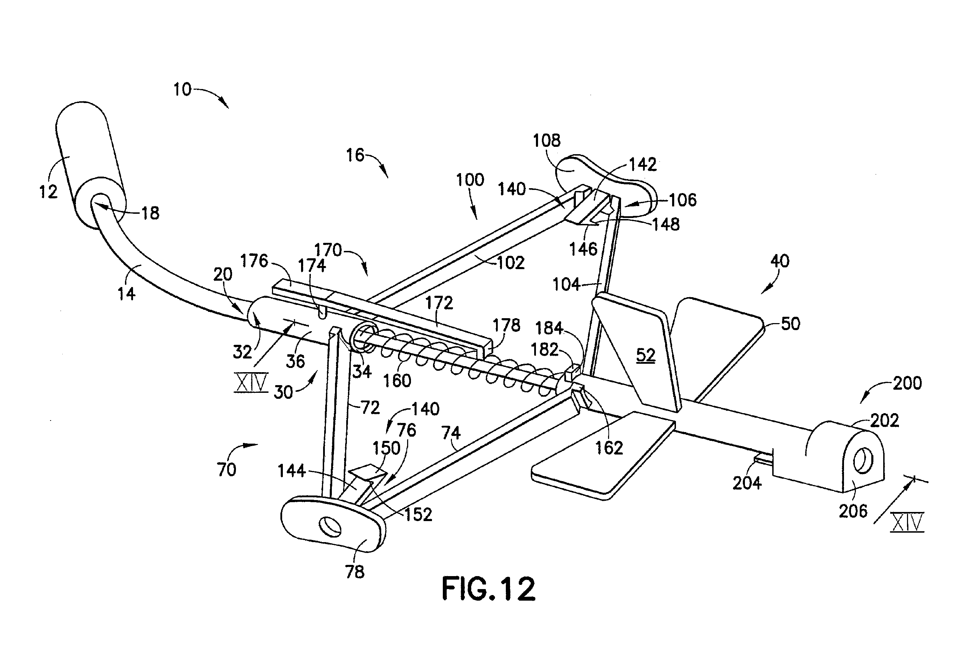

Referring to FIGS. 11-14, a further modification to the blood collection set **10** and needle assembly **16** of FIGS. 1-5 is shown. The blood collection set **10** and needle assembly **16** of FIGS. 11-15 is substantially similar to the blood collection set **10** and needle assembly **16** of FIGS. 1-5, but further includes a modified needle tip guard **200**. The tip guard **200** is generally comprised of a tip guard housing **202** and a protective clip **204**. The tip guard housing **202** is preferably a unitary structure molded from a thermoplastic material. The tip guard housing **202** may be formed integrally with the housing **42** of the shield member **40** as shown in FIGS. 11-14, or formed separately from and attached to the housing **42** of the shield member **40**. The tip guard housing **202** includes a distal end **206**, and an internal passage **208** extending through the tip guard housing **202** to cooperate with the central bore **44** of the shield member **40**. Portions of the internal passage **208** adjacent the distal end **206** define an enlarged clip receptacle or recess **210**. A clip mounting post **212** extends downward from the tip guard housing **202**. The protective clip **204** is preferably unitarily stamped and formed from a resiliently deflectable metallic material. The protective clip **204** includes a spring leg **214** with a proximal end **216** and an opposed distal end **218**. A mounting aperture (not shown) extends through the spring leg **214** at a location near the proximal end **216**. The mounting aperture has a diameter approximately equal to or slightly less than the diameter of the mounting post **212** of the tip guard housing **202**. As such, the mounting post **212** can be forced through the mounting aperture when the axis of the mounting post **212** and the axis of the mounting aperture are substantially co-linear. A lockout leg **220** extends angularly from the distal end **218** of the spring leg **214**. The lockout leg **220** is bent back toward the proximal end **216** of the spring leg **214**.

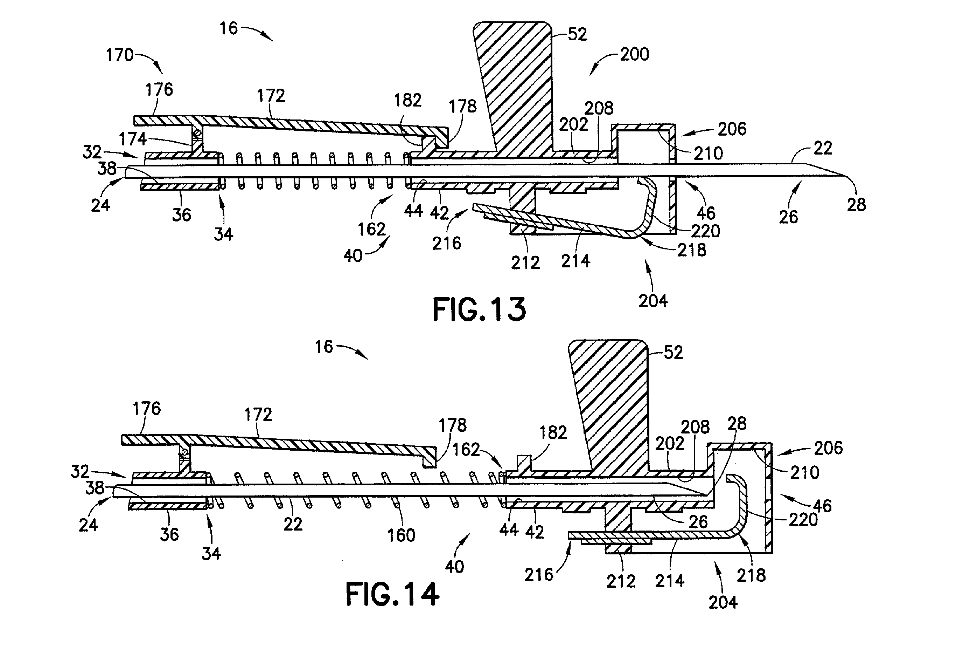

The modified tip guard **200** operates as follows. When the shield member **40** is in the retracted position, the lockout leg **220** is biased against the needle cannula **22** by the spring leg **214**, as shown in FIG. 13. When the latch assembly **170** is released of engagement between the hub member **30** and the shield member **40**, the compression spring **160** automatically (i.e., passively) begins to move the shield member **40** axially away from the hub member **30**, as discussed previously. Simultaneously, the needle cannula **22** is withdrawn into the housing **42** of the shield member **40**. During the movement of the shield member **40** axially away from the hub member **30**, the lockout leg **220** remains biased in contact with the needle cannula **22** by the spring leg **214**. As the shield member **40** reaches its fully extended position, the needle cannula **22** withdraws axially past the recess **210** formed by the tip guard housing **202**. Because the lockout leg **220** is spring-biased toward the recess **210** by the spring leg **214**, as soon as the needle cannula **22** is withdrawn past the lockout leg **220**, the lockout leg **220** is spring-biased by the spring leg **214** into the recess **210**. The lockout leg **220** thereby prevents re-emergence of the needle cannula **22** from the tip guard housing of the needle point guard.

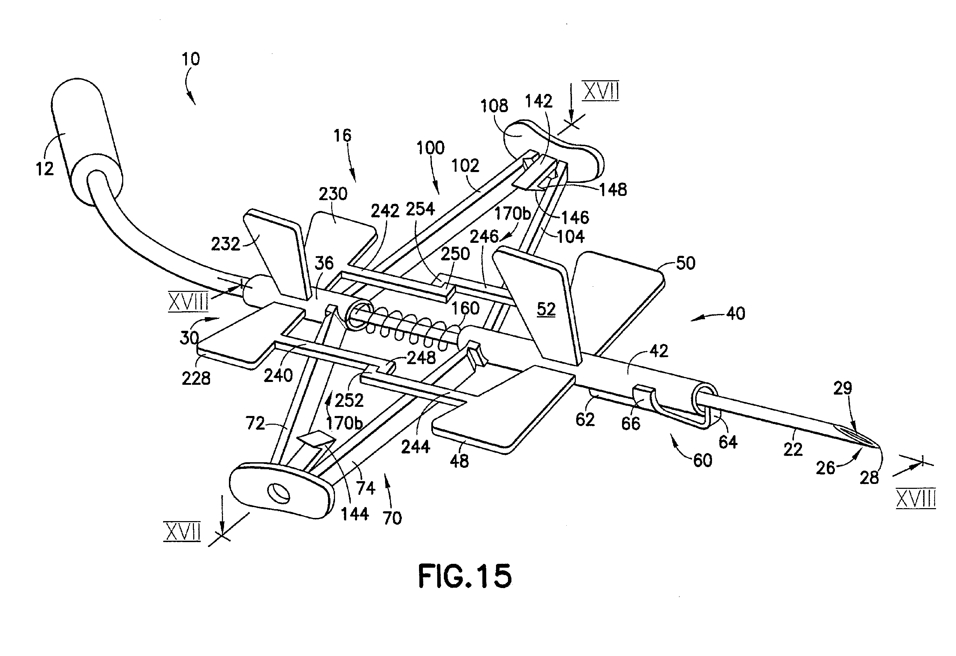

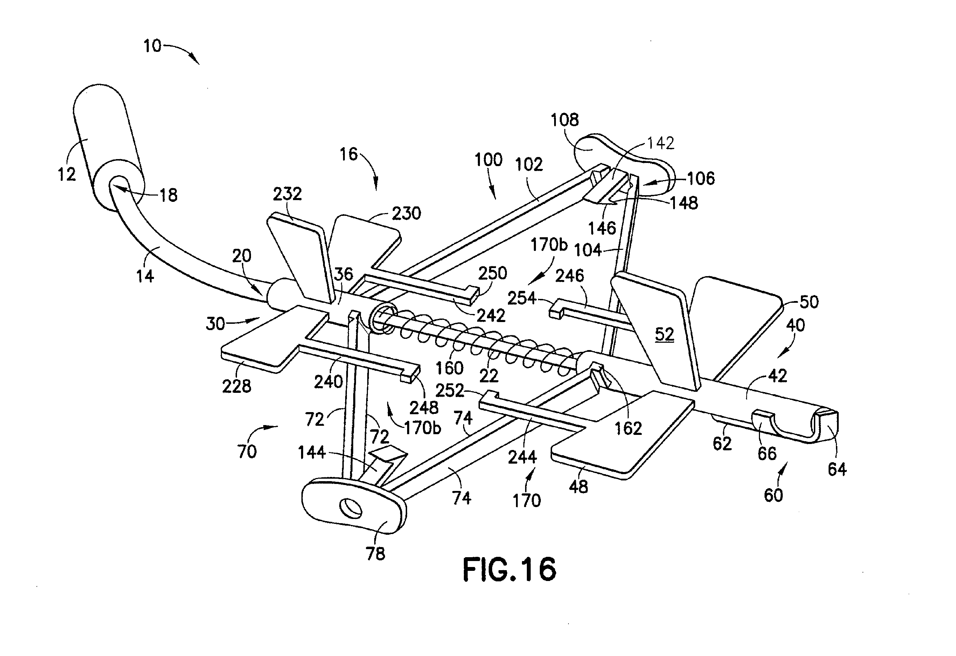

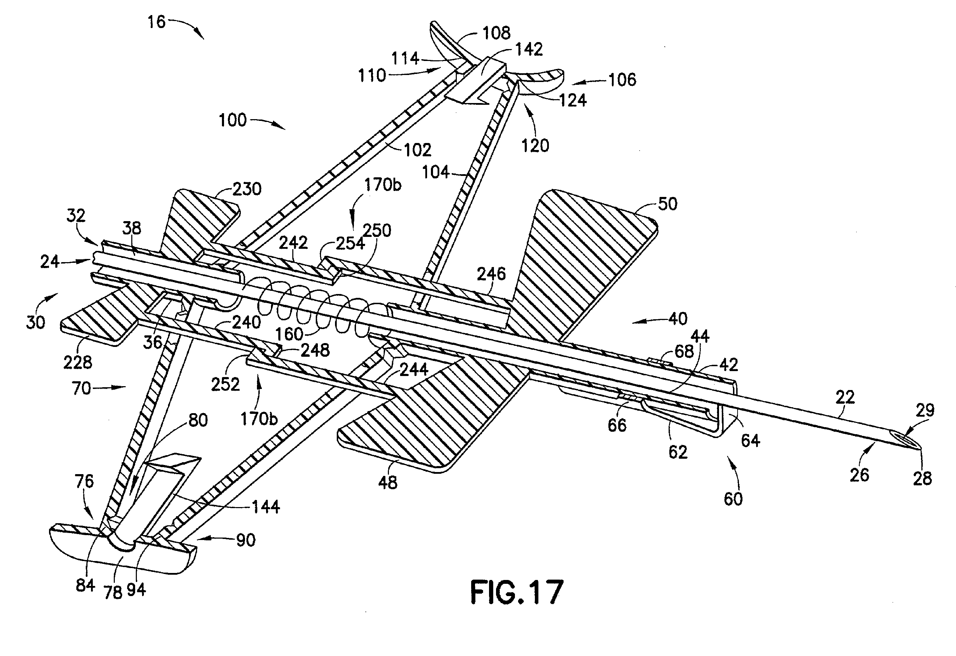

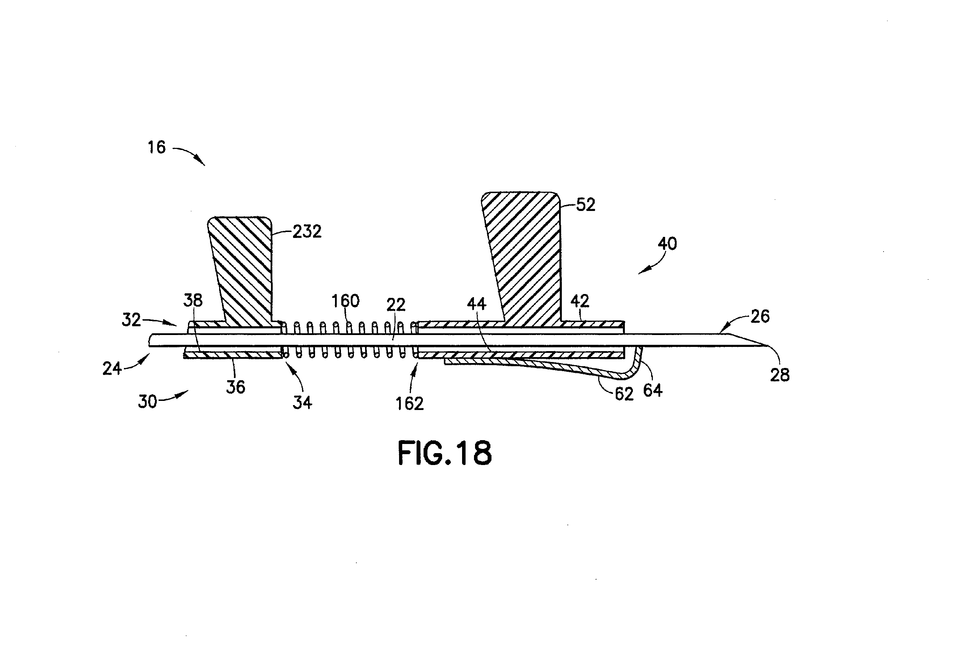

A still further modification of the blood collection set **10** and shieldable needle assembly **16** of the present invention is shown in FIGS. 15-18. The needle assembly **16** shown in FIGS. 15-18 is substantially similar to the needle assembly 16 discussed previously in connection with FIGS. 1-5 with two exceptions. First, the hub member **30** includes a pair of lateral wings **228**, **230** extending symmetrically outward from the rigid tubular wall **36** of the hub member **30.** A dorsal wing **232** may be located between the two lateral wings **228**, **230**. The dorsal wing **232** extends from a top surface of the hub member **30** and is located symmetrically between the lateral wings **228**, **230**. In addition, the lateral wings **228**, **230** and the dorsal wing **232** are preferably made of a resiliently flexible material so that lateral wings **228**, **230** and the dorsal wing **232** may be folded together in a manner similar to the lateral wings **48**, **50** and the dorsal wing **52** discussed previously.

A second modification to the blood collection set **10** and needle assembly **16** shown in FIGS. 15-18 is the location and orientation of the latch assembly. The latch assembly 170b is now comprised of a pair of latch members **240**, **242** located, respectively, on the lateral wings **228**, **230** extending from the hub member **30**. The latch members **240**, **242** extend toward the lateral wings **48**, **50** extending from the housing **42** of the shield member **40**. The latch members **240, 242** are configured to cooperate with a second pair of latch members **244**, **246** located, respectively, on the lateral wings **48**, **50** extending from the housing **42** of the shield member **40**. The latch members **244**, **246** located on the lateral wings **48**, **50** extending from the housing **42** of the shield member **40** extend proximally toward the lateral wings **228**, **230** extending from the hub member **30**. The latch members **240**, **242** include hooked portions **248**, **250** configured to mate with hooked portions **252**, **254** formed on the latch members **244**, **246**. The respective engagement of the hooked portions **248**, **250** with the hooked portions **252**, **254** maintains the shield member **40** in the retracted position, and prevents the compression spring **160** from moving the shield member **40** to the extended position.

The blood collection set **10** and assembly needle **16** shown in FIG. 15-18 may be used in a similar manner as the blood collection set **10** and needle assembly **16** discussed previously in connection with FIGS. 1-5. FIG. 15 shows the blood collection set **10** and needle assembly **16** with the shield member **40** in the retracted position. The shield member **40** is maintained in the retracted position through engagement between the respective hook portions **248**, **250** and **252**, **254** for the respective pairs of latch members **240**, **242** and **244**, **246**. The latch assembly **170b** is released of engagement between the hub member **30** and the shield member **40** by pivoting the lateral wings **48**, **50** extending from the shield member **40** or the lateral wings **228**, **230** extending from the hub member **30**. This releases the engagement between the respective hook portions **248**, **250** and **252**, **254** for the respective pairs of latch members **240**, **242** and **244**, **246**, which in turn releases the outward axial forces provided by the compression spring 160 to move the shield member **40** to the extended position.

Because the lateral wings **48, 50** extending outward from the shield member **40** and the lateral wings **228**, **230** extending outward from the hub member **30** are made of a resiliently flexible material, the latch members **240**, **242** extending from the lateral wings **228**, **230** may be easily disengaged from the latch members **244**, **246** extending from the lateral wings **48**, **50** by pivoting the lateral wings **228**, **230** upward or downward relative to the hub member **30** to release their engagement from the latch members **244**, **246**. A similar result occurs if the lateral wings **48**, **50** are pivoted upward or downward relative to the housing **42** of the shield member **40**. Additionally, the blood collection set **10** and needle assembly **16** of FIGS. 15-18 may include the tip guard **60** discussed in connection with FIGS. 1-6, or the tip guard **200** discussed in connection with FIGS. 11-14. FIGS. 15-18 illustrate the blood collection set **10** and needle assembly **16** with the tip guard **60**. In all other respects, the blood collection set **10** and the needle assembly **16** shown in FIGS. 15-18 are the same as the blood collection set **10** and needle assembly **16** discussed previously in connection with FIGS. 1-5.

Referring to FIGS. 19 and 20, a further modification to the blood collection set **10** and needle assembly **16** of FIGS. 1-5 is shown. In FIGS. 19 and 20, the compression spring **160a** of the needle assembly **16** is no longer located co-axially about the needle cannula **22**. The compression spring **160a** is now located adjacent the needle cannula **22**. The compression spring **160a** is connected to the shield member **40** and the hub member **30** in a similar manner as the compression spring **160a** discussed previously in connection with FIGS. 1-5. The latch member **172** of the latch assembly **170** is located on the hub member **30** and the catch member **182** of the latch assembly **172** is located on the shield member **40** in a similar manner as the latch assembly **170** of FIGS. 1-5. The locations for the latch member **172** and the catch member **182** may be reversed, as discussed previously. Additionally, the blood collection set **10** and needle assembly **16** of FIGS. 19 and 20 may include the tip guard **60** discussed in connection with FIGS. 1-6, or the tip guard **200** discussed in connection with FIGS. 11-14.

The needle assembly **16** of FIGS. 19 and 20 includes a modified locking assembly **280** extending from one of the fingerplates **78**, **108** toward the needle cannula **22**. In FIGS. 19 and 20, the locking assembly **280** is shown extending from the inside surface of fingerplate **108**. The modified locking assembly **280** is formed by a pair of locking members **282**, **284**. The locking members **282**, **284** each have sloped edges **286**, **288**, respectively at distal ends thereof, which face the needle cannula **22**. The locking members **282**, **284** further include transversely extending locking edges **290**, **292**, respectively, located opposite from the sloped edges **286**, **288**, respectively. The locking members **282**, **284** of the locking assembly **280** are preferably made of a resilient material such as molded plastic and are adapted to engage the needle cannula **22** when the shield member **40** is extended to the extended position by the compression spring **160**. In particular, when the latch member **172** of the latch assembly **170** is pivoted out of engagement with the catch member **182**, the compression spring **160a** located adjacent the needle cannula **22** causes the shield member **40** to move axially away from the hub member **30** until the shield member **40** reaches the extended position. Simultaneously, the extendable members **70**, **100** begin to move toward each other and toward the needle cannula **22**. As the fingerplate **108** carrying the locking assembly **280** moves toward the needle cannula **22**, the locking members **282**, **284** of the locking assembly **280** begins to engage the needle cannula **22**. In particular, the sloped edges **286**, **288** contact and slide over the needle cannula **22**, thereby separating the locking members **282**, **284** to receive the shaft of the needle cannula **22**. Eventually, the opposed locking members **282**, **284** fully engage around the needle cannula **22**, and the locking edges **290**, **292** prevent the needle cannula **22** from separating from the locking assembly **280**. The needle cannula **22** is now located between the locking members **252**, **254**. With the locking assembly **280** engaged around the needle cannula **22**, the extendable members **70, 100** are prevented from extending outward away from the lateral side of the needle assembly **16**, and the shield member **40** is prevented from moving from the extended position back to the retracted position adjacent the hub member **30**. The tip guard **60** shown in FIG. 19 will automatically cover the distal opening **46** of the central bore **44** of the housing **42** of the shield member **40** once the shield member **40** is moved over the puncture tip **28** of the needle cannula **22**. Similarly, the modified tip guard **200** shown in FIG. 20 will prevent the re-emergence of the puncture tip **28** of the needle cannula **22** once the shield member **40** and tip guard housing **202** have moved to the extended position covering the needle cannula **22**.

While the blood collection set and shieldable needle assembly of the present invention have been described with respect to preferred embodiments, various modifications and alterations of the present invention may be made without departing from the spirit and scope of the present invention. The scope of the present invention is defined in the appended claims and equivalents thereto.

## Claims

1. A shieldable needle assembly, comprising:
a needle cannula having a proximal end and a distal end with a puncture tip;
a hub member supporting the proximal end of the needle cannula;
a shield member having a housing defining a central bore, with the shield member in axial alignment with the hub member and extending co-axially about the needle cannula, and with the shield member movable between a retracted position in which the puncture tip of the needle cannula is exposed and an extended position in which the shield member covers the puncture tip of the needle cannula;
at least one extendable member connecting the hub member and the shield member;
a compression spring operatively connected between the hub member and the shield member; and
a latch assembly providing releasable engagement between the hub member and the shield member,
wherein when the latch assembly is in engagement between the hub member and the shield member the shield member is in the retracted position, and wherein when the latch assembly is released of engagement between the hub member and the shield member, the shield member is moved to the extended position by the compression spring, thereby covering the puncture tip of the needle cannula.

2. A needle assembly as in claim 1, wherein the latch assembly comprises a pivoting latch member located on one of the hub member and the shield member and configured to releasably engage with a catch member located on the other of the hub member and the shield member, such that when the latch member is pivoted out of engagement with the catch member, the latch assembly is released of engagement between the hub member and the shield member, thereby causing the shield member to be moved to the extended position by the compression spring.

3. A needle assembly as in claim 1, wherein the shield member includes a pair of butterfly wings extending laterally from opposing sides of the housing of the shield member.

4. A needle assembly as in claim 3, wherein the housing of the shield member further includes a dorsal wing located between the butterfly wings.

5. A needle assembly as in claim 1, wherein the hub member includes a pair of butterfly wings.

6. A needle assembly as in claim 5, wherein the hub member further includes a dorsal wing located between the butterfly wings.

7. A needle assembly as in claim 1, wherein the spring is located co-axially about the needle cannula.

8. A needle assembly as in claim 1, wherein the spring is located adjacent the needle cannula and extends between the hub member and the shield member.

9. A needle assembly as in claim 1, wherein the shield member includes a tip guard for protectively surrounding the puncture tip of the needle cannula when the shield member is moved to the extended position, and wherein the tip guard comprises a tip guard housing and a spring clip connected to the tip guard housing, with the spring clip biased against the needle cannula when the shield member is in the retracted position and resiliently extends over the puncture tip of the needle cannula when the shield member is moved to the extended position.

10. A needle assembly as in claim 1, further including a tip guard attached to the housing of the shield member and comprising a spring leg extending axially along the housing, a pair of clip legs securing the tip guard to the housing, and a blocking plate resiliently biased against the needle cannula when the shield member is in the retracted position and resiliently extends over a distal opening to the central bore of the housing when the shield member is moved of the extended position for preventing the puncture tip of the needle cannula from projecting from the shield member.

11. A needle assembly as in claim 1, wherein the extendable member is comprised of a pair of folding legs connected by a hinged knee joint, with one leg of the pair of legs hingedly connected to the hub member and the other leg of the pair of legs hingedly connected to the shield member.

12. A needle assembly as in claim 11, wherein the knee joint further includes a fingerplate to enable the user to manipulate the needle assembly.

13. A shieldable needle assembly as in claim 12, further including a locking assembly for locking the shield member in the extended position, with the locking assembly comprising a pair of locking members extending from the fingerplate toward the needle cannula and configured to engage the needle cannula such that when the shield member is moved to the extended position by the compression spring, the locking members engage the needle cannula to secure the shield member in the extended position.

14. A needle assembly as in claim 12, wherein the compression spring is located adjacent the needle cannula and extends between the hub member and the shield member, and further comprising a pair of locking members extending from the fingerplate toward the needle cannula and configured to engage the needle cannula such that when the shield member is moved to the extended position by the compression spring the locking members engage the needle cannula to secure the shield member in the extended position.

15. A needle assembly as in claim 1, further comprising a second extendable member connecting the hub member and the shield member and located opposite from the first extendable member, wherein the extendable members are each comprised of a pair of folding legs connected by a hinged knee joint, with one leg of the pair of legs for each extendable member hingedly connected to the hub member and the other leg of the pair of legs for each extendable member hingedly connected to the shield member.

16. A needle assembly as in claim 15, wherein the knee joint for each extendable member includes a fingerplate.

17. A needle assembly as in claim 16, further including a locking assembly for locking the shield member in the extended position, with the locking assembly comprising a locking member extending from one of the fingerplates toward the opposing fingerplate and configured to engage a second locking member extending from the opposing fingerplate such that when the shield member is moved to the extended position by the compression spring, the locking members engage to secure the shield member in the extended position.

18. A needle assembly as in claim 1, wherein the hub member, shield member, and extendable member are integrally molded as a one-piece unit of polymeric material.

19. A needle assembly as in claim 1, wherein the hub member is configured for connection to a flexible tube of a blood collection set.

20. A needle assembly as in claim 1, further including a removable needle cover positioned over the distal end of the needle cannula.

21. A shieldable blood collection set, comprising:
a flexible tube having opposed first and second ends, with the first end of the flexible tube adapted for connection to a receptacle;
a hub member mounted to the second end of the flexible tube;
a needle cannula having a proximal end and a distal end with a puncture tip, with the proximal end connected to the hub member and the distal end projecting from the hub member, and with the needle cannula defining a lumen in fluid communication with the flexible tube and the fixture;
a shield member having a housing defining a central bore, with the shield member in axial alignment with the hub member and extending co-axially about the needle cannula, and with the shield member movable between a retracted position in which the puncture tip of the needle cannula is exposed and an extended position in which the shield member covers the puncture tip of the needle cannula;
at least one extendable member connecting the hub member and the shield member;
a compression spring operatively connected between the hub member and the shield member; and
a latch assembly providing releasable engagement between the hub member and the shield member,
wherein when the latch assembly is in engagement between the hub member and the shield member the shield member is in the retracted position, and wherein when the latch assembly is released of engagement between the hub member and the shield member, the shield member is moved to the extended position by the compression spring, thereby covering the puncture tip of the needle cannula.

22. A shieldable needle assembly, comprising:
a needle cannula having a proximal end and a distal end with a puncture tip;
a body comprising a hub member supporting the proximal end of the needle cannula; a shield member having a housing defining a central bore, with the shield member in axial alignment with the hub member and extending co-axially about the needle cannula, and with the shield member movable between a retracted position in which the puncture tip of the needle cannula is exposed and an extended position in which the shield member covers the puncture tip of the needle cannula; and a pair of extendable folding legs connecting the hub member and the shield member and extending laterally along the needle assembly, with the pair of folding legs connected by a hinged knee joint, with one leg of the pair of legs hingedly connected to the hub member and the other leg of the pair of legs hingedly connected to the shield member;
a compression spring operatively connected between the hub member and the shield member and providing an axial outward force between the hub member and the shield member; and
a latch assembly providing releasable engagement between the hub member and the shield member,
wherein when the latch assembly is in engagement between the hub member and the shield member, the shield member is in the retracted position, and wherein when the latch assembly is released of engagement between the hub member and the shield member, the shield member is moved to the extended position by the compression spring, thereby covering the puncture tip of the needle cannula.

23. A needle assembly as in claim 22, wherein both the hub member and the shield member include a pair of pivoting butterfly wings extending laterally from opposing sides thereof.

24. A needle assembly as in claim 23, wherein the shield member further includes a dorsal wing located between the butterfly wings.

25. A needle assembly as in claim 23, wherein the latch assembly comprises a pair of latch members located, respectively, on the wings extending from the shield member and configured to engage releasably with a second pair of latch members located, respectively, on the wings extending from the hub member, and wherein when the respective pairs of latch members are engaged the shield member is in the retracted position, and when one of the pairs of wings extending from the hub member and shield member are pivoted, the engaged latch members are disengaged and the shield member is moved to the extended position by the compression spring, thereby covering the puncture tip of the needle cannula.

26. A needle assembly as in claim 22, wherein the knee joint for the pair of folding legs includes a fingerplate.

27. A needle assembly as in claim 26, further including a locking assembly for locking the shield member in the extended position, with the locking assembly comprising a pair of locking members extending from the fingerplate toward the needle cannula and configured to engage the needle cannula such that when the shield member is moved to the extended position by the compression spring, the locking members engage the needle cannula to secure the shield member in the extended position.

28. A needle assembly as in claim 22, further comprising a second pair of extendable folding legs connecting the hub member and the shield member and located opposite from the first pair of extendable legs, wherein the second pair of extendable legs is connected by a hinged knee joint, with one leg of the second pair of extendable legs hingedly connected to the hub member and the other leg of the second pair of extendable legs hingedly connected to the shield member, and wherein the knee joint for the first and second pairs of extendable legs has a fingerplate.

29. A needle assembly as in claim 28, further including a locking assembly for locking the shield member in the extended position, with the locking assembly comprising a locking member extending from one of the fingerplates toward the opposing fingerplate and configured to engage a second locking member extending from the opposing fingerplate such that when the shield member is moved to the extended position by the compression spring, the locking members engage to secure the shield member in the extended position.

30. A method for automatically placing a shieldable needle assembly into a safety state in which a puncture tip of a needle cannula is covered by the needle assembly, comprising the steps of:
providing a shieldable needle assembly, comprising:
a needle cannula having a proximal end and a distal end with a puncture tip;
a hub member supporting the proximal end of the needle cannula;
a shield member having a housing defining a central bore, with the shield member in axial alignment with the hub member and extending co-axially about the needle cannula, and with the shield member movable between a retracted position in which the puncture tip of the needle cannula is exposed and an extended position in which the shield member covers the puncture tip of the needle cannula;
at least one extendable member connecting the hub member and the shield member;
a compression spring operatively connected between the hub member and the shield member; and
a latch assembly providing releasable engagement between the hub member and the shield member, with the latch assembly comprising a pivoting latch member located on one of the hub member and the shield member and engaged with a catch member located on the other of the hub member and the shield member;
using the needle assembly in a medical procedure; and
pivoting the latch member out of engagement with the catch member such that the shield member is moved to the extended position by the compression spring, thereby covering the puncture tip of the needle cannula.
